# EUROPÄISCHE PATENTANMELDUNG

(11) **EP 0 878 470 A1**
(43) Veröffentlichungstag der Anmeldung: **18.11.1998**
(21) Anmeldenummer: 98106826.5
(22) Anmeldetag: 15.04.1998
(51) Int. Cl.: C07D 301/12

(54) **Verfahren zur Herstellung von Epoxiden aus Olefinen**

(30) Priorität: 18.04.1997 DE 19716412
(71) Anmelder: Linde Aktiengesellschaft, 65189 Wiesbaden (DE)
(72) Erfinder: Ponceau, Marianne, Dipl.-Ing. (FH), 80687 München (DE); Müller-Markgraf, Wolfgang, Dr. Dipl.-Chem., 80689 München (DE)

(57) **Zusammenfassung**

Verfahren zur Herstellung von Epoxiden aus Olefinen, wobei mit Hilfe eines Oxidationsgases in einer Oxidationsstufe die Epoxidierung der Olefine mit Hilfe von Wasserstoffperoxid über einem geeigneten Epoxidationskatalysator in einer Arbeitslösung, die Alkylanthrahydrochinon und Alkylanthrachinon als Redoxsystem, einen Chinonlöser und einen Hydrochinonlöser und außerdem einen niedrig siedenden Alkohol enthält, vorgenommen wird und eine Erzeugung des Wasserstoffperoxids, in-situ oder in einem der Epoxidierung vorgeschalteten separaten Arbeitsschritt, durch Oxidation des Alkylanthrahydrochinons zu Alkylanthrachinon erfolgt. Erfindungsgemäß wird der Arbeitslösung zur Erzeugung des Wasserstoffperoxids und der Epoxide ein Stabilisator zugesetzt.

## Beschreibung

Die Erfindung betrifft ein Verfahren zur Herstellung von Epoxiden aus Olefinen, wobei mit Hilfe eines Oxidationsgases in einer Oxidationsstufe die Epoxidierung der Olefine mit Hilfe von Wasserstoffperoxid über einem geeigneten Epoxidationskatalysator in einer Arbeitslösung, die Alkylanthrahydrochinon und Alkylanthrachinon als Redoxsystem, einen Chinonlöser und einen Hydrochinonlöser und außerdem einen niedrig siedenden Alkohol enthält, vorgenommen wird und eine Erzeugung des Wasserstoffperoxids, in-situ oder in einem der Epoxidierung vorgeschalteten separaten Arbeitsschritt, durch Oxidation des Alkylanthrahydrochinons zu Alkylanthrachinon erfolgt.

Aus dem United States Patent No. 5,221,795 ist bekannt, daß es möglich ist, Epoxide aus Olefinen durch Reaktion mit Sauerstoff oder Luft in Anwesenheit eines Titan-Silicalit-Katalysators und eines Redoxsystems bestehend aus Alkylanthrachinon/Alkylanthrahydrochinon in einem geeigneten Lösungsmittelgemisch herzustellen. Dazu wird zunächst das Alkylanthrachinon in einer Lösung aus z.B. Methylnaphthalin (Chinonlöser) und Diisobutylketon (Hydrochinonlöser) über einem Hydrierkatalysator, z.B. einem Palladiumkatalysator, unter Einsatz von Wasserstoff zum Alkylanthrahydrochinon hydriert. Anschließend wird der Hydrierkatalysator abgetrennt und das Olefin zugesetzt. Im nächsten Schritt wird mit Hilfe von Sauerstoff oder Luft eine Oxidation des Alkylanthrahydrochinons zum Alkylanthrachinon unter Freisetzung von Wasserstoffperoxid durchgeführt. Dieses setzt sich mit dem Olefin über einem Titan-dotierten Zeolithkatalysator, z.B. vom Ti-ZSM-5 Typ zum entsprechenden Epoxid um. Olefinkomponenten der Kettenlängen 2 bis 18 können eingesetzt werden. Die nunmehr oxidierte "Arbeitslösung", die nun Anthrachinon enthält, wird in die Hydrierstufe zurückgeführt, dort erneut hydriert und wiederum der Epoxidationsstufe zugeführt.

Es handelt sich hierbei um eine Kombination eines bekannten Verfahrens zur Herstellung von Wasserstoffperoxid mit einem ebenfalls bekannten Verfahren zur Epoxidation von Olefinen über Ti-Zeolithkatalysatoren mit Wasserstoffperoxid. Die Epoxidation von z.B. Propylen mit Wasserstoffperoxid über Ti-haltigen Katalysatoren ist beispielsweise aus US Patent 5,354,875 bekannt.

Eine Kombination der zwei Verfahrensschritte mit einer Wasserstoffperoxidproduktion in-situ bei gleichzeitiger Epoxidation in nur einem Reaktionsgefäß bietet den Vorteil, daß kein Wasserstoffperoxid eingesetzt werden muß, sondern lediglich Wasserstoff (für die Hydrierstufe) und Sauerstoff oder Luft (für die Wasserstoffperoxiderzeugung/Epoxidation) als Einsatzstoffe benötigt werden. Vorausgesetzt, daß die Ausbeuten über beide Verfahrensschritte ausreichend hoch sind, wirkt sich dies günstig auf die Wirtschaftlichkeit der Epoxidation aus.

Aus US-Patent Nr. 5,463,090 ist ferner eine Variante des Verfahrens bekannt, in der die Erzeugung des Wasserstoffperoxids in einer vorgeschalteten separaten Stufe erfolgt, und erst in einer zweiten nachgeschalteten Stufe über einem Titan-Silicalit-Katalysator das Olefin epoxidiert wird. Zur Erhöhung der Löslichkeit des Redoxsystems in polar-protischen Lösungsmitteln wie Wasser und niederen Alkoholen wurde hier das Alkylanthrachinon/Alkylanthrahydrochinonsystem dahingehend variiert, daß die Alkylammoniumsalze der Sulfonsäure-substituierten Anthrachinone bzw. Hydrochinone zum Einsatz kommen.

Beim bisherigen Stand der Technik nach US-Patent 5,221,795 ist zu beobachten, daß bei der in-situ Wasserstoffperoxiderzeugung und Epoxidation von z.B. Propylen viel geringere Ausbeuten an Propylenoxid erzielt werden als aufgrund der eingesetzten Mengen Alkylanthrachinon und dem daraus erzeugten Wasserstoffperoxid erwartet werden kann. Obgleich nach US-Patent 5,221,795 die Propylenoxidausbeute bezogen auf das eingesetzte Ethylanthrahydrochinon mit bis zu 78 % angegeben wird, läßt sich dies in keinem Fall reproduzieren. (Eine Vergleichbarkeit ist sowieso nicht gegeben, da aus dieser Patentschrift nichts über die Ausbeute der Hydrierstufe bekannt ist, so daß damit zu rechnen ist, daß die Ausbeuten in Bezug auf den eigentlichen Einsatzstoff, nämlich das Ethylanthrachinon, deutlich geringer sind.)

In eigenen Versuchen zur Verifizierung von US-Patent 5,221,795 wurden über einem Ti-ZSM-5 Katalysator unter Bedingungen, die mit denen in den Ausführungsbeispielen der Patentschrift vergleichbar waren, in typischen Fällen nur ca. 5 % Propylenoxid-Ausbeute bezogen auf das eingesetzte Ethylanthrachinon gefunden. Mit einer derart geringen Ausbeute wird das Verfahren unwirtschaftlich.

Aufgabe der Erfindung ist es daher, das Verfahren aus dem US-Patent 5,221,795 hinsichtlich der Ausbeuten zu verbessern.

Diese Aufgabe wird erfindungsgemäß gelöst von einem Verfahren mit den Merkmalen des Anspruchs 1. Ausführungen der Erfindung sind Gegenstand von Unteransprüchen.

Kennzeichnend an der Erfindung ist, daß der Arbeitslösung zur Erzeugung des Wasserstoffperoxids und der Epoxide ein Stabilisator zugesetzt wird.

Es hat sich nämlich gezeigt, daß unter den Bedingungen, wie sie bei einem Verfahren nach dem Stand der Technik vorliegen und beispielsweise in US 5,221,795 beschrieben sind, das epoxidierende Agens, nämlich Wasserstoffperoxid, schon während seiner Bildung gleichzeitig rasch wieder zersetzt wird. Diese Zersetzung findet auf einer Zeitskala statt, die vergleichbar oder sogar kürzer ist, als die für die Epoxidierungsreaktion notwendige Verweilzeit. Das bedeutet, daß der spontane Zerfall von Wasserstoffperoxid kinetisch stark mit der Epoxidierung eines hinzugegebenen Olefins, z.B. Propylen, konkurriert. Als zwangsläufige Konsequenz werden die Epoxidausbeuten stark herabgesetzt. Bekannt ist, daß Spuren von Metallen in der Lösung zu einer Zersetzung des Wasserstoffperoxids führen. Da jedoch die Arbeitslösung im ersten Schritt immer mit Hilfe eines Palladium- oder Nickelkatalysators hydriert werden muß (Bildung des Alkylanthrahydrochinons aus dem Alkylanthrachinon), können Spuren von Palladium oder Nickel (oder eines beliebigen anderen Metalls, das sich zum Hydrieren eignet) prinzipiell nicht ausgeschlossen werden. Zwar wird der Hydrierkatalysator vor dem Oxidationsschritt abgetrennt, aber es kann nie ausgeschlossen werden, daß Spuren in der Lösung verbleiben. Mit diesem Nachteil ist insbesondere deshalb zu rechnen, weil stets die Möglichkeit besteht, daß geringe Mengen des Metalls kolloidal in Lösung gehen und daher nicht mehr filtrierbar sind. Der Stabilisator, der erfindungsgemäß der Arbeitslösung zugesetzt wird, beseitigt diesen Nachteil.

Vorteilhafterweise kann mindestens eine Metall- oder Metalloxid-komplexierende oder -bindende Substanz als Stabilisator in der Arbeitslösung suspendiert oder gelöst werden.

Eine oder mehrere der folgenden Substanzen werden in der Arbeitslösung als Stabilisator suspendiert oder gelöst:
- Phosphorsäure
- Alkaliphosphate oder -hydrogenphosphate, wie Dinatrium(kalium)hydrogenphosphat, Natrium(kalium)dihydrogenphosphat.
- Dinatriumdihydrogenpyrophosphat
- Tetranatriumdiphosphat
- Ammoniumphosphate und -hydrogenphosphate
- Ammoniumnitrat
- Nitrilotriessigsäure (NTA) oder deren Alkalisalze
- Ethylendinitriloessigsäure (EDTA) oder deren Alkalisalze
- Triethylentetraminhexaessigsäure (TTHA)
- (N-(2-Hydroxyethyl)-ethylendiamin-N,N,N'-triessigsäure Trinatriumsalz
- Ethylenglycol-bis(β-aminoethylether)-N,N,-tetraessigsäure (EGTA)
- 3-Aza-3-(carboxymethyl)-pentamethylendinitrilotetraessigsäure (DTPA)
- 1,2-Cyclohexandiamin-N,N,N,N'-tetraessigsäure.

Diese Substanzen sind geeignet, einzeln oder in Kombination Metalle in elementarer oder ionischer Form zu komplexieren oder in sonstiger Form zu binden. Die Erfindung ist jedoch nicht auf die angegebenen Substanzen beschränkt. Entscheidend ist ihre Eigenschaft, die bei der Anwendung vorliegenden Metalle zu binden.

Vorteilhaft kann eine Konzentration jeder einzelnen Substanz des Stabilisators in der Arbeitslösung zwischen 0.001 Gew.% und 10 Gew.%, bevorzugt zwischen 0.01 und 1 Gew.%, besonders bevorzugt aber eine Konzentration gewählt werden, die die Löslichkeitsgrenze in der Arbeitslösung nicht überschreitet.

Vorteilhaft ist es, wenn die Arbeitslösung als Chinonlöser, einen alkylsubstituierten Ein- oder Mehrkernaromaten, bevorzugt 1- oder 2-Methylnaphthalin, enthält sowie zusätzlich als Hydrochinonlöser einen polaren Kohlenwasserstoff aus der Gruppe der Ketone oder Ester, Diisobutylketon und außerdem als niedrig siedenden Alkohol Methanol.

Günstigerweise werden die Konzentrationsverhältnisse von Chinonlöser, Hydrochinonlöser und Alkohol so eingestellt, daß es möglich ist, die Substanzen des Stabilisators innerhalb der in Anspruch 4 genannten Konzentrationsbereiche zu lösen.

In einer günstigen Ausführung der Arbeitslösung werden 1 bis 10 Gew.%, bevorzugt 2 bis 5 Gew.%, Alkylanthrachinon gelöst.

In einer bevorzugten Ausführungsform wird die Alkylanthrachinon enthaltende Arbeitslösung mit dem Stabilisator zunächst mit Hilfe eines geeigneten Hydrierkatalysators, bevorzugt eines Palladium- oder Nickelkatalysators, bei Wasserstoff-Drucken zwischen 1 und 10 bar, bevorzugt 2 bis 5 bar, und Temperaturen von 10 bis 100°C, bevorzugt bei 20 bis 60°C, zu Alkylanthrahydrochinon hydriert und die so entstandene Alkylanthrahydrochinonlösung nach Abtrennung des Hydrierkatalysators der Oxidationsstufe zugeführt.

Bevorzugt wird in der Oxidationsstufe die den Stabilisator und das Alkylanthrahydrochinon enthaltende Arbeitslösung in Anwesenheit von mindestens stöchiometrischen Mengen Olefin oder von Olefin im Überschuß mit Luft oder Reinsauerstoff bei Drucken zwischen 1 und 20 bar, bevorzugt zwischen 2 und 5 bar, und Temperaturen zwischen 0 und 80°C, bevorzugt zwischen 20 und 60°C, oxidiert.

In einer günstigen Ausführung des erfindungsgemäßen Verfahrens werden die Olefine, beispielsweise Ethylen oder Propylen, im Überschuß in der Arbeitslösung gelöst und anschließend das Oxidationsgas, bevorzugt Luft oder Sauerstoff, unverdünnt oder in beliebiger Stickstoffverdünnung zugesetzt.

Der nicht abreagierte Überschuß an gasförmigen Olefinen kann vorteilhafterweise in die den Stabilisator enthaltende oxidierte Arbeitslösung zurückgeführt werden.

Die Zugabe des Oxidationsgases erfolgt zweckmäßigerweise so, daß die Gaskonzentrationen über der Arbeitslösung außerhalb der Explosionsgrenzen bleiben.

Bevorzugt wird in der Oxidationsstufe ein Epoxidationskatalysator eingesetzt, der nicht durch die Stabilisatorsubstanzen deaktiviert wird, bevorzugt ein Titan-dotierter Silicalit aus einer der Gruppen TS-1, TS-2, ZSM-11, ZSM-12, ZSM-48, Faujasit oder mesoporöser MCM-41, besonders bevorzugt ein Titan-dotierter Zeolith-Katalysator vom TS-1 (= ZSM-5) Typ.

Der Epoxidationskatalysator kann mit weiteren Metallen als Promotoren dotiert sein.

In bevorzugten Ausführungen der Erfindung wird die Konzentration des Epoxidationskatalysators in der Arbeitslösung so eingestellt, daß die Reaktion bei Verweilzeiten zwischen 10 Minuten und 5 Stunden, bevorzugt 20 Minuten und 1,5 Stunden, vollständig abläuft.

Das erfindungsgemäße Verfahren kann vorteilhaft zur Herstellung von Epoxiden mit zwei oder drei Kohlenstoffatomen, bevorzugt zur Herstellung von Ethylenoxid, angewendet werden.

Die Erfindung wird anhand von zwei auf Versuchen fußenden Beispielen und einer Figur zu Beispiel 1 erläutert.

### Beispiel 1

Dieses Beispiel veranschaulicht die Wirkung der Stabilisatoren bei der Bildung von Wasserstoffperoxid in der Arbeitslösung.

### 1. Hydrierung

In einem 80 ml Autoklav werden 1,8 g 2-Ethylanthrachinon in 37 ml Lösungsmittel vorgelegt. Das Lösungsmittel besteht aus 17,5 Vol.% 2-Methylnaphthalin, 56 Vol.% Diisobutylketon und 26,5 Vol.% Methanol. Als Hydrierkatalysator wird 0,2 g Palladium auf Aktivkohleträger hinzugefügt. Anschließend wird bei 50°C und 4 bar Wasserstoff unter Rühren hydriert. Man erhält die 2-Ethylanthrahydrochinonlösung durch Abfiltrieren des Katalysators unter Stickstoffatmosphäre.

### 2. Oxidation, Erzeugung von Wasserstoffperoxid

Die 2-Ethylanthrahydrochinonlösung wird im ersten Ansatz bei Atmosphärendruck mit Luft bei Raumtemperatur oxidiert und der Gehalt an Wasserstoffperoxid wird in Abhängigkeit von der Zeit gemessen (Jodometrische Titration mit Natriumthiosulfat). Im zweiten Ansatz wird genauso vorgegangen, nur wird die 2-Ethylanthrahydrochinonlösung vor der Oxidation mit 0.02 Gew.% Phosphorsäure versetzt und mit einem Gemisch aus Ammoniumnitrat und Dinatriumhydrogenphosphat aufgesättigt. Die überschüssigen Salze werden abgetrennt und die Lösung bei Atmosphärendruck oxidiert. Die Wasserstoffperoxidkonzentration über der Zeit wird gemessen.

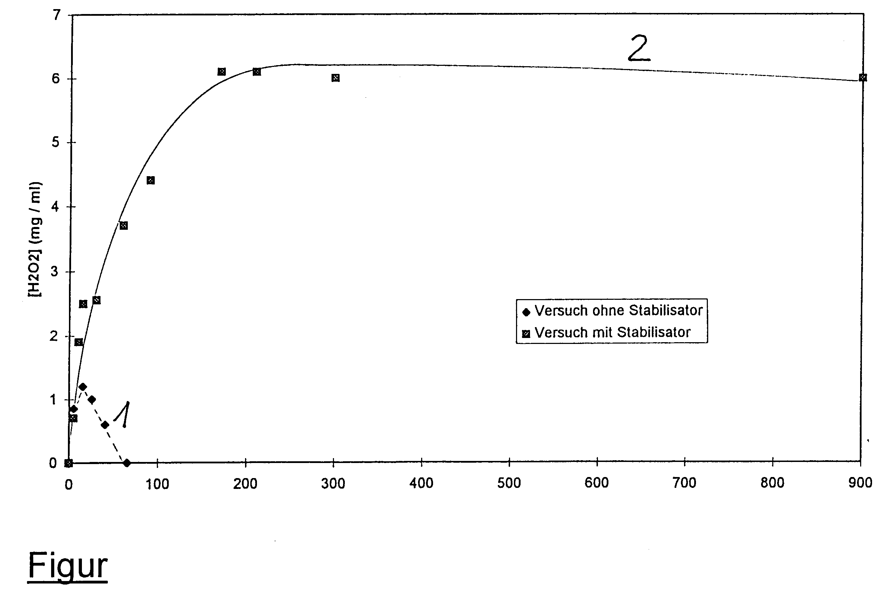

Die Ergebnisse sind in der Figur für die Fälle mit und ohne Stabilisatorbehandlung dargestellt. Es wird deutlich, daß der Maximalwert der Wasserstoffperoxidkonzentration im Fall ohne Stabilisatorzusatz, Kurve 1 in der Figur, weit unterhalb der stationären Endkonzentration im Fall mit Stabilisatorzusatz, Kurve 2 in der Figur, liegt. Darüber hinaus zerfällt das Wasserstoffperoxid im ersten Fall innerhalb einer Zeitspanne (ca. 60 Minuten) die kurz gegenüber der notwendigen Verweilzeit des Epoxidationsschrittes ist (bei Raumtemperatur wären mehr als ca. 3 Stunden notwendig). Es steht somit kaum noch Wasserstoffperoxid für die Epoxidation zur Verfügung.

### Beispiel 2

Anhand dieses Beispiels wird die Epoxidierung von Propylen mit in-situ Erzeugung von Wasserstoffperoxid nach dem erfindungsgemäßen Verfahren erläutert.

Es wird eine 2-Ethylanthrahydrochinonlösung durch Hydrierung wie in Beispiel 1 hergestellt und die Lösung wird wie in Beispiel 1 beschrieben mit der dort angegebenen Stabilisatormischung versetzt. Von dieser Lösung werden 34 ml in einem 80 ml Autoklaven vorgelegt und es werden insgesamt 16 bar Propylen aufgedrückt (13 bar bleiben gelöst und 3 bar stellen sich als Restdruck ein). Anschließend wird mit Luft bei 2 bar unter Rühren bei 50°C epoxidiert. Nach 90 Minuten Reaktionszeit erhält man 90 % Ausbeute an Propylenoxid bezogen auf das ursprünglich eingesetzte 2-Ethylanthrachinon.

Zum Vergleich wurde ein völlig identischer Ansatz, jedoch ohne Stabilisatorzusatz, unter gleichen Bedingungen untersucht. Hier betrug die Ausbeute an Propylenoxid lediglich 5 %.

## Patentansprüche

1. Verfahren zur Herstellung von Epoxiden aus Olefinen, wobei mit Hilfe eines Oxidationsgases in einer Oxidationsstufe die Epoxidierung der Olefine mit Hilfe von Wasserstoffperoxid über einem geeigneten Epoxidationskatalysator in einer Arbeitslösung, die Alkylanthrahydrochinon und Alkylanthrachinon als Redoxsystem, einen Chinonlöser und einen Hydrochinonlöser und außerdem einen niedrig siedenden Alkohol enthält, vorgenommen wird und eine Erzeugung des Wasserstoffperoxids, in-situ oder in einem der Epoxidierung vorgeschalteten separaten Arbeitsschritt, durch Oxidation des Alkylanthrahydrochinons zu Alkylanthrachinon erfolgt, **dadurch gekennzeichnet**, daß der Arbeitslösung zur Erzeugung des Wasserstoffperoxids und der Epoxide ein Stabilisator zugesetzt wird.

2. Verfahren nach Anspruch 1, **dadurch gekennzeichnet**, daß als Stabilisator mindestens eine Metall- oder Metalloxid-komplexierende oder -bindende Substanz in der Arbeitslösung suspendiert oder gelöst wird.

3. Verfahren nach Anspruch 1 oder 2, **dadurch gekennzeichnet**, daß eine oder mehrere der folgenden Substanzen in der Arbeitslösung als Stabilisator suspendiert oder gelöst werden:
- Phosphorsäure
- Alkaliphosphate oder -hydrogenphosphate, wie Dinatrium(kalium)hydrogenphosphat, Natrium(kalium)dihydrogenphosphat.
- Dinatriumdihydrogenpyrophosphat
- Tetranatriumdiphosphat
- Ammoniumphosphate und -hydrogenphosphate
- Ammoniumnitrat
- Nitrilotriessigsäure (NTA) oder deren Alkalisalze
- Ethylendinitriloessigsäure (EDTA) oder deren Alkalisalze
- Triethylentetraminhexaessigsäure (TTHA)
- (N-(2-Hydroxyethyl)-ethylendiamin-N,N,N'-triessigsäure Trinatriumsalz
- Ethylenglycol-bis(β-aminoethylether)-N,N,-tetraessigsäure (EGTA)
- 3-Aza-3-(carboxymethyl)-pentamethylendinitrilotetraessigsäure (DTPA)
- 1,2-Cyclohexandiamin-N,N,N,N'-tetraessigsäure.

4. Verfahren nach einem der Ansprüche 1 bis 3, **dadurch gekennzeichnet**, daß eine Konzentration jeder einzelnen Substanz des Stabilisators in der Arbeitslösung zwischen 0.001 Gew.% und 10 Gew.%, bevorzugt zwischen 0.01 und 1 Gew.%, besonders bevorzugt aber eine Konzentration gewählt wird, die die Löslichkeitsgrenze in der Arbeitslösung nicht überschreitet.

5. Verfahren nach einem der Ansprüche 1 bis 4, **dadurch gekennzeichnet**, daß die Arbeitslösung als Chinonlöser, einen alkylsubstituierten Ein- oder Mehrkernaromaten, bevorzugt 1- oder 2-Methylnaphthalin, enthält sowie zusätzlich als Hydrochinonlöser einen polaren Kohlenwasserstoff aus der Gruppe der Ketone oder Ester, Diisobutylketon und außerdem als niedrig siedenden Alkohol Methanol.

6. Verfahren nach einem der Ansprüche 1 bis 5, **dadurch gekennzeichnet**, daß die Konzentrationsverhältnisse von Chinonlöser, Hydrochinonlöser und Alkohol so eingestellt werden, daß es möglich ist, die Substanzen des Stabilisators innerhalb der in Anspruch 4 genannten Konzentrationsbereiche zu lösen.

7. Verfahren nach einem der Ansprüche 1 bis 6, **dadurch gekennzeichnet**, daß in der Arbeitslösung 1 bis 10 Gew.%, bevorzugt 2 bis 5 Gew.%, Alkylanthrachinon gelöst sind.

8. Verfahren nach einem der Ansprüche 1 bis 7, **dadurch gekennzeichnet**, daß die Alkylanthrachinon enthaltende Arbeitslösung mit dem Stabilisator zunächst mit Hilfe eines geeigneten Hydrierkatalysators, bevorzugt eines Palladium- oder Nickelkatalysators, bei Wasserstoff-Drucken zwischen 1 und 10 bar, bevorzugt 2 bis 5 bar, und Temperaturen von 10 bis 100°C, bevorzugt bei 20 bis 60°C, zu Alkylanthrahydrochinon hydriert und die so entstandene Alkylanthrahydrochinonlösung nach Abtrennung des Hydrierkatalysators der Oxidationsstufe zugeführt wird.

9. Verfahren nach einem der Ansprüche 1 bis 8, **dadurch gekennzeichnet**, daß in der Oxidationsstufe die den Stabilisator und das Alkylanthrahydrochinon enthaltende Arbeitslösung in Anwesenheit von mindestens stöchiometrischen Mengen Olefin oder von Olefin im Überschuß mit Luft oder Reinsauerstoff bei Drucken zwischen 1 und 20 bar, bevorzugt zwischen 2 und 5 bar, und Temperaturen zwischen 0 und 80°C, bevorzugt zwischen 20 und 60°C, oxidiert wird.

10. Verfahren nach einem der Ansprüche 1 bis 9, **dadurch gekennzeichnet**, daß die Olefine, beispielsweise Ethylen oder Propylen, im Überschuß in der Arbeitslösung gelöst werden und anschließend das Oxidationsgas, bevorzugt Luft oder Sauerstoff, unverdünnt oder in beliebiger Stickstoffverdünnung zugesetzt wird.

11. Verfahren nach einem der Ansprüche 1 bis 10, **dadurch gekennzeichnet**, daß der nicht abreagierte Überschuß an gasförmigen Olefinen in die den Stabilisator enthaltende oxidierte Arbeitslösung zurückgeführt wird.

12. Verfahren nach einem der Ansprüche 1 bis 11, **dadurch gekennzeichnet**, daß die Zugabe des Oxidationsgases so erfolgt, daß die Gaskonzentrationen über der Arbeitslösung außerhalb der Explosionsgrenzen bleiben.

13. Verfahren nach einem der Ansprüche 1 bis 12, **dadurch gekennzeichnet**, daß in der Oxidationsstufe ein Epoxidationskatalysator eingesetzt wird, der nicht durch die Stabilisatorsubstanzen deaktiviert wird, bevorzugt ein Titan-dotierter Silicalit aus einer der Gruppen TS-1, TS-2, ZSM-11, ZSM-12, ZSM-48, Faujasit oder mesoporöser MCM-41, besonders bevorzugt ein Titan-dotierter Zeolith-Katalysator vom TS-1 (= ZSM-5) Typ.

14. Verfahren nach einem der Ansprüche 1 bis 13, **dadurch gekennzeichnet**, daß der Epoxidationskatalysator mit weiteren Metallen als Promotoren dotiert sein kann.

15. Verfahren nach einem der Ansprüche 1 bis 14, **dadurch gekennzeichnet**, daß die Konzentration des Epoxidationskatalysators in der Arbeitslösung so eingestellt wird, daß die Reaktion bei Verweilzeiten zwischen 10 Minuten und 5 Stunden, bevorzugt 20 Minuten und 1,5 Stunden vollständig abläuft.

16. Anwendung des Verfahrens nach einem der Ansprüche 1 bis 15 zur Herstellung von Epoxiden mit zwei oder drei Kohlenstoffatomen, bevorzugt zur Herstellung von Ethylenoxid.
